(19) **Europäisches Patentamt / European Patent Office / Office européen des brevets**

(11) **EP 4 563 993 A1**

(12) **EUROPEAN PATENT APPLICATION**
published in accordance with Art. 153(4) EPC

(43) Date of publication:
04.06.2025 Bulletin 2025/23

(21) Application number: 23846616.3

(22) Date of filing: 27.07.2023

(51) International Patent Classification (IPC):
*G01N 27/22* (2006.01)    *G01N 27/00* (2006.01)
*G01N 27/06* (2006.01)

(52) Cooperative Patent Classification (CPC):
G01N 27/00; G01N 27/06; G01N 27/22

(86) International application number:
PCT/JP2023/027567

(87) International publication number:
WO 2024/024886 (01.02.2024 Gazette 2024/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.07.2022 JP 2022121000

(71) Applicants:
• KYB Corporation
Minato-ku,
Tokyo 105-5128 (JP)
• National Institute of Technology
Hachioji-shi, Tokyo 1930834 (JP)

(72) Inventors:
• YOSHIDA, Takahiro
Tokyo 105-5128 (JP)
• KAMEDA, Yukinori
Tokyo 105-5128 (JP)
• DEGUCHI, Mikio
Niihama-shi, Ehime 792-8580 (JP)

(74) Representative: Grünecker Patent- und
Rechtsanwälte
PartG mbB
Leopoldstraße 4
80802 München (DE)

(54) **FLUID CHARACTERISTIC DETECTION DEVICE**

(57)    The fluid-property detection device includes: the resonant circuit (8) formed by electrically connecting the capacitor (C1) and the inductor portion (3101) to each other, the capacitor (C1) being formed by the ground electrode and detection electrode (21) immersed in the fluid; the excitation-signal oscillator portion (3102) configured to excite the resonant circuit (8) with the excitation signal having a predetermined oscillation frequency; and the control unit (3112) configured to control the oscillation frequency such that the phase difference between the excitation signal and the output signal from the resonant circuit (8) maintains a predetermined phase difference, wherein the fluid property is detected through the change in the oscillation frequency.

FIG.4

EP 4 563 993 A1

**Description**

TECHNICAL FIELD

The present invention relates to a fluid-property detection device.

BACKGROUND ART

There is a known technique of outputting, to a resonant circuit including an inductor and a capacitor, which is formed of a ground electrode and a detection electrode immersed in a fluid, an excitation signal having a frequency close to the resonance frequency of the resonant circuit and monitoring a fluid properties on the basis of a phase difference between the excitation signal and an output signal from the resonant circuit (Japanese Unexamined Utility Model Registration Application Publication No. 61-170064).

SUMMARY OF INVENTION

However, with the above-described technique, the sensitivity for detecting the phase difference is deteriorated as the phase difference is changed, and therefore, it is difficult to sensitively monitor the fluid property in subsequent measurements.

The present invention has been made in view of the above-described problems, and an object of the present invention is to provide a fluid-property detection device capable of performing monitoring for sensitively detecting a change in a fluid property.

A fluid-property detection device according to one embodiment of the present invention is a fluid-property detection device including a resonant circuit including a ground electrode configured to be immersed in a fluid, a detection electrode configured to be immersed in the fluid and arranged so as to face the ground electrode, and an inductor portion connected to the detection electrode, the resonant circuit being formed by electrically connecting a capacitor and the inductor portion to each other, the capacitor being formed by the ground electrode and the detection electrode, an excitation-signal oscillator portion configured to excite the resonant circuit with an excitation signal having a predetermined oscillation frequency, a phase difference detector configured to detect a phase difference between the excitation signal and an output signal from the resonant circuit, and a control unit configured to control the oscillation frequency such that the phase difference maintains a predetermined phase difference, wherein the fluid-property detection device detects the fluid property through change in the oscillation frequency.

BRIEF DESCRIPTION OF DRAWINGS

[FIG. 1] FIG. 1 is a perspective view of the fluid-property detection device according to a first embodiment.
[FIG. 2] FIG. 2 is a sectional view of the fluid-property detection device according to the first embodiment.
[FIG. 3] FIG. 3 is a sectional view taken along a line A-A in FIG. 2.
[FIG. 4] FIG. 4 is a circuit diagram of the fluid-property detection device according to the first embodiment.
[FIG. 5] FIG. 5 is a circuit diagram of an inductor portion forming the fluid-property detection device according to the first embodiment.
[FIG. 6] FIG. 6 is a diagram for explaining a gain and a phase difference detected by the fluid-property detection device according to the first embodiment.
[FIG. 7] FIG. 7 is a control flow of the fluid-property detection device according to the first embodiment.
[FIG. 8] FIG. 8 is a circuit diagram of the fluid-property detection device according to a second embodiment.
[FIG. 9] FIG. 9 is a sectional view of an inner electrode, a detection electrode, and a cover electrode that form the fluid-property detection device according to a third embodiment.

DESCRIPTION OF EMBODIMENTS

In the following, this embodiment will be described with reference to the drawings.

[Basic Configuration of Fluid-Property Detection Device according to First Embodiment]

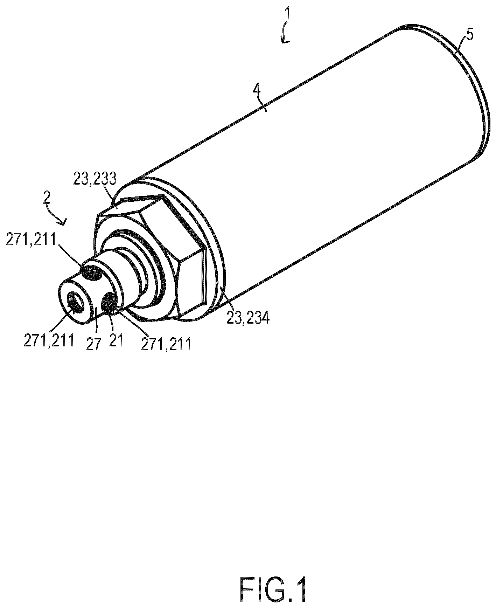

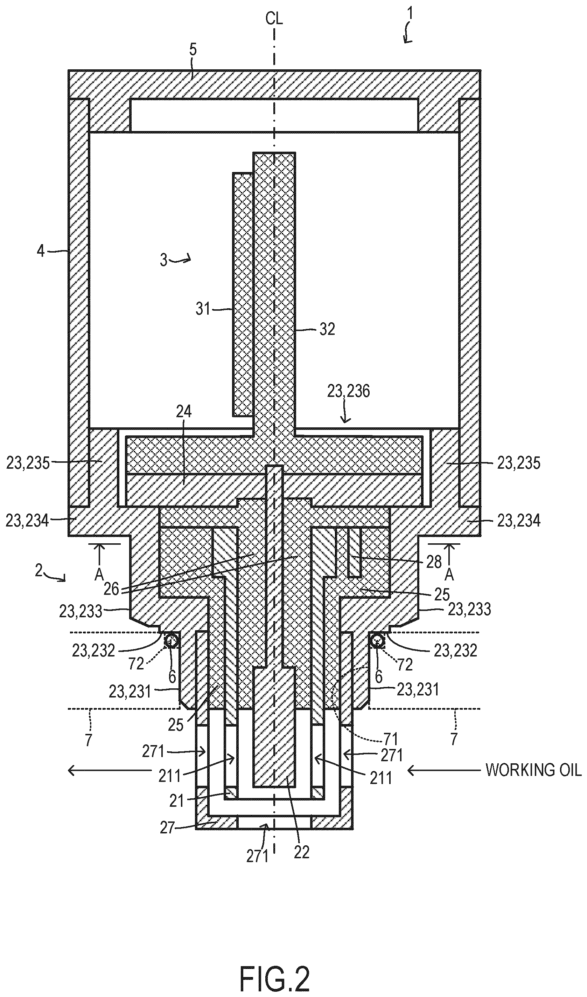

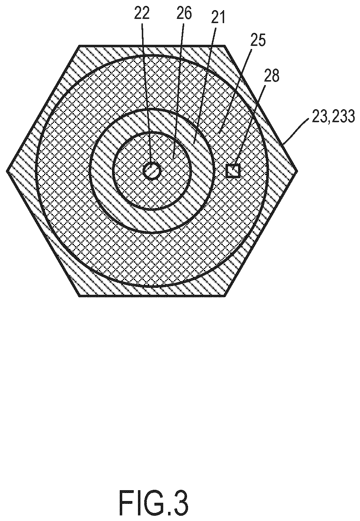

FIG. 1 is a perspective view of a fluid-property detection device 1 according to this embodiment. FIG. 2 is a sectional view of the fluid-property detection device 1 of this embodiment. FIG. 3 is a sectional view taken along a line A-A in FIG. 2.

As shown in FIGs. 1 and 2, the fluid-property detection device 1 has a detection unit 2, a substrate unit 3 that is attached to the detection unit 2, a cylindrical cover 4 having a cylindrical shape that is attached to the detection unit 2 and covers the substrate unit 3, and an end cover 5 that closes an opening of the cylindrical cover 4.

As shown in FIG. 2, the fluid-property detection device 1 (the detection unit 2, the substrate unit 3, the cylindrical cover 4, and the end cover 5) has a cylindrical shape centered on a center axis CL (a concentric shape).

The fluid-property detection device 1 is designed to detect, for example, a property of a working oil serving as a working fluid that is contained in a hydraulic device (not show) such as a hydraulic cylinder. The fluid-property detection device 1 is attached to a predetermined position of a container, such as a pipe 7, a tank, or the like, that is provided on the hydraulic device and contains the working oil. Note that, an inspection target of the fluid-property detection device 1 is not limited to the working oil, and it may be various liquids and gases such as lubricating oil, cutting oil, mineral oil, fuel, solvent, chemical agents, and so forth. The fluid-property detection device 1 may be applied without being limited to the viscosity of the fluid. In the following, a description will be given of a case in which the fluid-property detection device 1 detects the property of the working oil in the pipe 7 (see FIG. 2) as a detection target fluid.

In the following, the direction parallel to the center axis CL is referred to as the axial direction of the fluid-property detection device 1, and an the direction orthogonal to the axial direction is referred to as the radial direction of the fluid-property detection device 1. In addition, the side at which the end cover 5 is arranged is referred to as the base end side of the fluid-property detection device 1, and the side at which the detection unit 2 is arranged is referred to as the tip end side of the fluid-property detection device 1.

The detection unit 2 has a detection electrode 21 having a tubular shape, an inner electrode 22 (a ground electrode) having a rod shape that is arranged on the center axis CL inside the detection electrode 21, a housing 23 that is attached to the pipe 7 of the hydraulic device (not shown), and an metal attachment plate 24 serving as a fixing member that holds the inner electrode 22 and that is fixed to the housing 23 by a screw, etc.

The detection unit 2 is provided with: an outer spacer 25 that is provided between the housing 23 and the detection electrode 21 to insulates the housing 23 from the detection electrode 21, and defines the position of the detection electrode 21 relative to the housing 23; and an inner spacer 26 that is provided between the detection electrode 21 and the inner electrode 22 to insulates the detection electrode 21 from the inner electrode 22, and defines the positions of the detection electrode 21 and the inner electrode 22 relative to the housing 23.

As shown in FIG. 3 (which is a sectional view taken along a line A-A in FIG. 2), the inner electrode 22, the inner spacer 26, the detection electrode 21, the outer spacer 25, and the housing 23 are arranged in this order in the direction in which the diameter increases with the center axis CL as the center. The inner spacer 26, the detection electrode 21, the outer spacer 25, and the housing 23 each has a ring shape. Note that, the inner spacer 26 and the outer spacer 25 may be constructed as a single unit by integral molding.

The detection unit 2 has an excitation electrode 28 at a position facing the detection electrode 21 in the radial direction on the tip end side of the housing 23. As shown in FIGs. 2 and 3, the excitation electrode 28 has a rod shape and is arranged at a position away from the detection electrode 21 in the radial direction by a predetermined distance, and is electrically insulated from the detection electrode 21 with respect to direct current. In addition, a side surface of the excitation electrode 28 and a surface of the excitation electrode 28 in the axial direction, which is the surface on the tip end side of the housing 23, are supported by the outer spacer 25. In addition, a surface of the excitation electrode 28 on the base end side is supported by the inner spacer 26.

The detection unit 2 is attached to an opening of the housing 23 and has a metal cover electrode 27 (a ground electrode) that covers tip end portions of the detection electrode 21 and the inner electrode 22.

The housing 23 is made of a metal material having an electrically conductive property, and has an attachment portion 231, a flange portion 232, a nut portion 233, a cover receiving portion 234, and a cover insertion portion 235 in this order from the tip end side.

The attachment portion 231 has a male screw structure and is screwed to an attachment hole 71 (a female screw) formed in the pipe 7. An annular recessed portion 72 is arranged at a portion of the attachment hole 71 of the pipe 7 that comes into contact with the housing 23 (the flange portion 232), and an O-ring 6 is arranged in the recessed portion 72. The flange portion 232 is formed at a base portion of the attachment portion 231, and the flange portion 232 forms the surface that faces the recessed portion 72 (the O-ring 6).

Thus, by screwing the attachment portion 231 into the attachment hole 71, the O-ring 6 is compressed between the flange portion 232 and a bottom surface of the recessed portion 72, and thereby, a space between the pipe 7 and the housing 23 is sealed.

As shown in FIGs. 1 and 3, the nut portion 233 is formed to have a hexagonal columnar shape. Therefore, by rotating the nut portion 233 using a tool, such as a spanner, it is possible to easily attach the attachment portion 231 to the attachment hole 71.

The cover insertion portion 235 is a portion that has a cylindrical shape and that is inserted into the cylindrical cover 4. The cover receiving portion 234 is a flange portion that protrudes from the base portion of the nut portion 233 in the radial direction, and a tip end surface of the cylindrical cover 4 comes into contact with the cover receiving portion 234.

The cover insertion portion 235 is inserted into the cylindrical cover 4, and in a state in which the tip end surface of the cylindrical cover 4 is in contact with the cover receiving portion 234, the cylindrical cover 4 is fixed to the cover insertion portion 235 by a screw, etc.

The end cover 5 is fixed to the cylindrical cover 4 by a screw, etc. in a state in which an opening of the cylindrical cover 4 on the base end side is closed. The substrate unit 3 is accommodated in an accommodation space defined by the end cover 5, the cylindrical cover 4, and the detection unit 2 (the housing 23).

The base end side of the housing 23, which is a portion positioned on inner side of the cover insertion portion 235, forms attachment plate accommodation portion 236, and the attachment plate 24 is attached to the attachment plate accommodation portion 236.

The substrate unit 3 has a circuit board 31 and a circuit board holding member 32 that holds the circuit board 31. The circuit board holding member 32 is fixed to the attachment plate 24 of the detection unit 2 by a screw, etc.

The circuit board 31 has wiring (not shown) that is individually connected to the housing 23, the detection electrode 21, and the excitation electrode 28.

Here, the housing 23 is directly connected to the pipe 7. In addition, the cover electrode 27 made of metal is connected to the housing 23. The inner electrode 22 is electrically connected to the housing 23 via the attachment plate 24. Thus, when the pipe 7 is grounded, the housing 23, the cover electrode 27, and the inner electrode 22 are also grounded.

In addition, if the cylindrical cover 4 and the end cover 5 are made of metal, the cylindrical cover 4 and the end cover 5 are also grounded. With such a configuration, the accommodation space is electrostatically shielded, and the circuit board 31 is protected from electrical disturbance.

As shown in FIG. 2, at least a portion of the cover electrode 27 is immersed in the working oil. Flow holes 271 are formed in the portion of the cover electrode 27 that is immersed in the working oil (a bottom surface, and a side surface), and with such a configuration, at least a portion of the detection electrode 21 is immersed in the working oil. In addition, flow holes 211 are also formed in the portion of the detection electrode 21 that is immersed in the working oil (a bottom surface, and a side surface), and with such a configuration, at least a portion of the inner electrode 22 is immersed in the working oil.

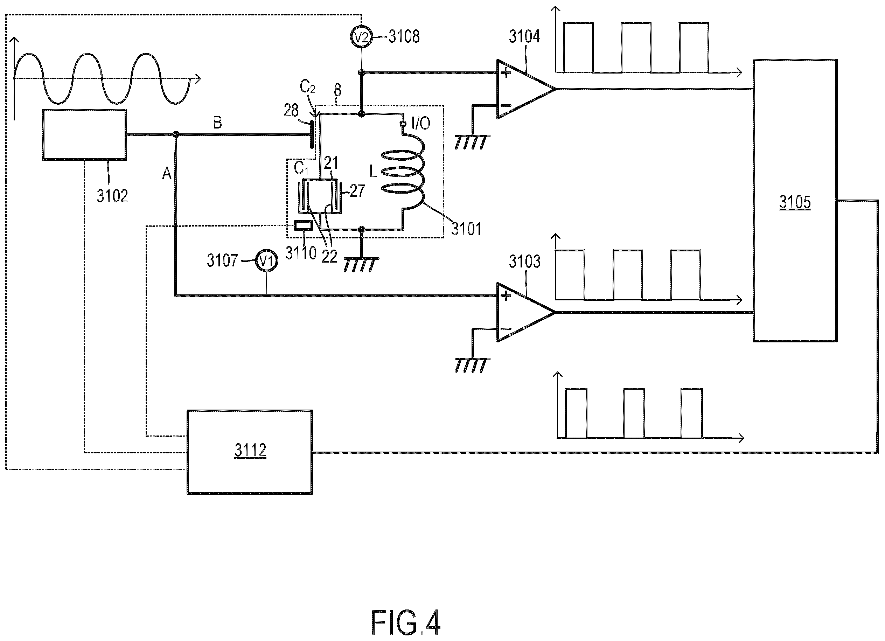

The cover electrode 27, the detection electrode 21, and the inner electrode 22 are positioned so as to be separated from each other with predetermined gaps, and the working oil flows through between these gaps. With such a configuration, a capacitor C1, in which the working oil serving as a dielectric material is interposed between the cover electrode 27, the detection electrode 21, and the inner electrode 22, is formed (see FIG. 4, where the capacitance is from several pF to 10 pF, for example). By electrically connecting the cover electrode 27, the detection electrode 21, and the inner electrode 22 to an inductor portion 3101, which will be described later, a resonant tank that forms a resonant circuit 8, which will be described later, is constructed.

In addition, the detection electrode 21 and the excitation electrode 28 are separated from each other by the outer spacer 25 that is positioned therebetween. With such a configuration, a capacitor C2 (parasitic capacitance) (see FIG. 3), in which the outer spacer 25 serving as the dielectric material is sandwiched between the detection electrode 21 and the excitation electrode 28, is formed.

Note that a circuit portion of the substrate unit 3 other than the inductor portion 3101 (see FIG. 4), which will be described later, does not need to be attached to the housing 23, and it may be possible to employ a configuration in which the circuit portion is arranged away from the pipe 7 and is electrically connected to the detection electrode 21, the inner electrode 22, the cover electrode 27, and the excitation electrode 28 via the wiring. With such a configuration, it is possible to ensure waterproofness of the substrate unit 3 and prevent a short circuit.

[Circuit Diagram]

FIG. 4 is a circuit diagram of the fluid-property detection device 1 according to this embodiment. On the circuit board 31 of the fluid-property detection device 1 (see FIG. 2), the inductor portion 3101, an excitation-signal oscillator portion 3102, a first comparator 3103, a second comparator 3104, a phase comparator 3105, a first voltage sensor 3107, a second voltage sensor 3108, a temperature sensor 3110, and a control unit 3112 are arranged.

In addition, the circuit forming the fluid-property detection device 1 has a first circuit A, in which an excitation signal reaches the first comparator 3103 from the excitation-signal oscillator portion 3102 as a starting point, and a second circuit B, in which the excitation signal reaches the second comparator 3104 from the excitation-signal oscillator portion 3102 as the starting point via the capacitor C2 (the excitation electrode 28 and the detection electrode 21), and the resonant circuit 8, which will be described later, is connected to the second circuit B.

As described above, the capacitor C1 is formed by the detection electrode 21, together with the inner electrode 22 and the cover electrode 27 (the ground electrode), and has a structure in which the working oil is sandwiched therebetween. In the capacitor C1, the detection electrode 21 side is connected to a non-inverting input terminal (+) of the second comparator 3104 and to an input/output terminal I/O of the inductor portion 3101, which will be described later, and the inner electrode 22 side and the cover electrode 27 side (the ground electrode side) are grounded. In the above, the ground electrode refers to the entire circuit region that has a substantially zero potential (ground potential) with respect to an AC signal component.

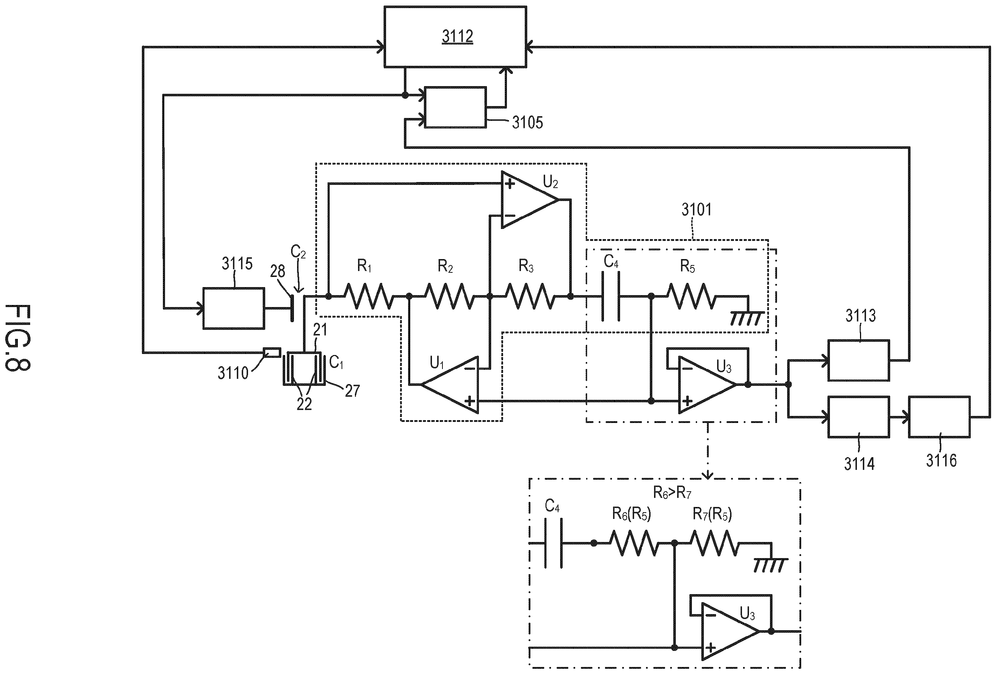

The capacitor C2 is formed by the excitation electrode 28 and the detection electrode 21. Note that, in a first embodiment, although the excitation-signal oscillator portion 3102 outputs a sinusoidal excitation signal, when the excitation signal includes a harmonic component, a low-pass filter (a first low-pass filter 3115 shown in FIG. 8) is arranged between the excitation-signal oscillator portion 3102 and the excitation electrode 28.

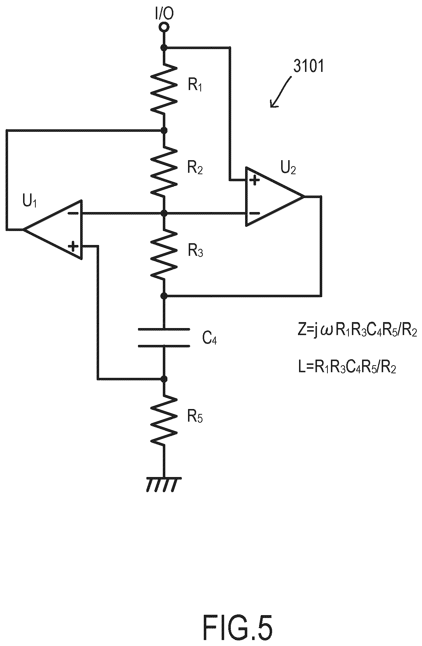

One end of the inductor portion 3101 serves as the input/output terminal I/O (see FIG. 5), which will be described later, and it is connected to the capacitor C1 and the non-inverting input terminal (+) of the second comparator 3104. On the other hand, the other end of the inductor portion 3101 is grounded. Thus, the inductor portion 3101 forms, together with the capacitor C1, the parallel resonant circuit 8. In this case, the resonance frequency f0 of the resonant circuit 8 is expressed by the following equation (1).

Equation (1):

$$f_0 = \frac{1}{2\pi\sqrt{LC_1}} \qquad \cdots(1)$$

In addition, another resonance frequency f1, which characterizes a frequency characteristic of the intensity of the signal excited by the resonant circuit 8, appears as the resonance frequency f1 of the resonant circuit 8 that is electrically connected to the capacitor C2, and it is expressed by the following equation (2).
Equation (2):

$$f_1 = \frac{1}{2\pi\sqrt{L(C_1+C_2)}} \qquad \cdots(2)$$

The intensity of the output signal to be excited by the resonant circuit 8 reaches a maximum value between these two resonance frequencies. Because the capacitor C2 is the parasitic capacitance formed between the excitation electrode 28 and the detection electrode 21, the capacitor C2 is sufficiently smaller than the capacitor C1. Therefore, these two resonance frequencies f0 and f1 have very close values.

As shown in FIG. 5, for example, the inductor portion 3101 is a GIC (General Impedance Converter) circuit in which an operational amplifier U1 and an operational amplifier U2 are connected to a series circuit, in which a resistor R1, a resistor R2, a resistor R3, a capacitor C4, and a resistor R5 are connected in this order. In other words, the inductor portion 3101 is an equivalent inductor (a virtual inductor) that is configured by an impedance component that does not include an inductor. A first end of the series circuit (on the resistor R1 side) serves as the input/output terminal I/O of the inductor portion 3101, and a second end of the series circuit (on the resistor R5 side) is grounded. In the above, the term "grounded" means a connection to a substantially zero potential (a ground potential) with respect to the AC signal component.

In the operational amplifier U1, the non-inverting input terminal (+) is connected to the connection midpoint between the capacitor C4 and the resistor R5, and inverting input terminal (-) is connected to the connection midpoint between the resistor R2 and the resistor R3, and an output terminal is connected to the connection midpoint between the resistor R1 and the resistor R2.

In the operational amplifier U2, the non-inverting input terminal (+) is connected to the input/output terminal I/O, the inverting input terminal (-) is connected to the connection midpoint between the resistor R2 and the resistor R3, and the output terminal is connected to the connection midpoint between the resistor R3 and the capacitor C4.

In the above-described configuration, the impedance Z of the inductor portion 3101 as seen from the input/output terminal I/O is given by Z = j ω(R1 R3 C4 R5)/(R2), and the inductance L is given by (R1 R3 C4 R5)/(R2). Here, if R2 and R3 are set to the same resistance value, the inductance L can be set to a large value by setting C4, R1, and R5 to large values, and thereby, it is possible to set the resonance frequencies f0 and f1 to low values (for example, several kHz).

Note that, it is preferable for the inductance L of the inductor portion 3101 to be approximately 100 [H], for example, and in this case, for example, it is preferable to set C4 to 0.01 [μF], R1 to 1 [MΩ], R5 to 10 [kΩ], and R2 to 1 [kΩ].

As shown in FIG. 4, the excitation-signal oscillator portion 3102 outputs the excitation signal having a predetermined oscillation frequency f to the excitation electrode 28 (the capacitor C2) and the first comparator 3103. In addition, the excitation-signal oscillator portion 3102 can change the oscillation frequency f on the basis of a control signal that is input from the control unit 3112. As the excitation-signal oscillator portion 3102, an oscillation circuit capable of generating a sinusoidal wave of a relatively low frequency (about several kHz), such as a CR oscillation circuit, is applied. Note that, in a second embodiment described below, a square wave is applied as the excitation signal.

In the first comparator 3103 (a first square wave generator), the excitation signal is input to a non-inverting input terminal (+) and the inverting input terminal (-) is grounded. The first comparator 3103 compares the magnitude relationship between the voltage of the excitation signal and the reference voltage, and for example, outputs a first square wave that becomes 1 (High) when the voltage of the excitation signal is equal to or higher than the reference voltage and that becomes 0 (Low) when the voltage of the excitation signal is lower than the reference voltage.

In the second comparator 3104 (a second square wave generator), the output signal (the voltage) of the resonant circuit 8 is input to the non-inverting input terminal (+), and the inverting input terminal (-) is grounded. The second comparator 3104 compares the magnitude relationship between the output signal (the voltage) of the resonant circuit 8 and the reference voltage, and for example, outputs a second square wave that becomes 1 (High) when the output signal (the voltage) is equal to or higher than the reference voltage and that becomes 0 (Low) when the voltage of the excitation signal is lower than the reference voltage.

The phase comparator 3105 outputs a third square wave, which is the logical product of the first square wave and the second square wave, to the control unit 3112. In other words, the third square wave becomes 1 (High) when the first square wave is 1 (High) and the second square wave is 1 (High) and becomes 0 (Low) in all other cases. Thus, when there is no time difference (phase difference) between the first square wave and the second square wave, the pulse width of the third square wave matches the widths of the first square wave and the second square wave, but when there is a time difference (a phase difference), the pulse width is decreased as the time difference is increased.

Note that, even if the third square wave generated by the phase comparator 3105 is a logical OR or an exclusive OR of the first square wave and the second square wave, the third square wave has functionally equivalent effects although the relationship between the increase and decrease of the phase difference and the increase and decrease of the pulse width generated is different. Alternatively, equivalent effects can also be achieved by using a circuit that utilizes an edge-triggered flip-flop to generate the third square wave in accordance with the time difference between the rising edges or the falling edges of the first square wave and the second square wave.

The first voltage sensor 3107 detects the excitation signal (the voltage) (V1) and outputs it to the control unit 3112.

The second voltage sensor 3108 detects the output signal (the voltage) of the resonant circuit 8 (V2) and outputs it to the control unit 3112.

The temperature sensor 3110 detects the temperature of the working oil (the detection electrode 21, the inner electrode 22, and the cover electrode 27) and outputs it to the control unit 3112. In addition, when the detection electrode 21 and the inductor portion 3101 are arranged adjacent to each other and the temperature difference between them is small, the temperature sensor 3110 detects the temperature of the inductor portion 3101 as the temperature of the working oil and outputs it to the control unit 3112.

The control unit 3112 is configured by, for example, a microcomputer, and drives the fluid-property detection device 1 (a fluid-property detection method) according to this embodiment in accordance with a predetermined program.

The voltage (V1) of the excitation signal is input to the control unit 3112 from the first voltage sensor 3107, and the voltage (V2) of the output signal from the resonant circuit 8 is input to the control unit 3112 from the second voltage sensor 3108.

The third square wave is input to the control unit 3112 from the phase comparator 3105. The control unit 3112 detects the phase difference between the excitation signal and the output signal from the resonant circuit 8 by detecting rising time and falling time of the third square wave that has been input from the phase comparator 3105, and the control unit 3112 controls the excitation-signal oscillator portion 3102 (the oscillation frequency f) such that the phase difference becomes a predetermined phase difference ($\theta0$ (for example $\pi/2$)). Here, as the time difference between the rising time and the falling time of the third square wave becomes shorter, the phase difference between the excitation signal and the output signal from the resonant circuit 8 becomes larger, and conversely, the phase difference becomes smaller as the time difference becomes longer.

Note that, it may be possible to employ a configuration in which the third square wave that is output from the phase comparator 3105 is smoothed by the low-pass filter (not shown), and the phase difference between the excitation signal and the output signal from the resonant circuit 8 is calculated by the control unit 3112 on the basis of the magnitude of the DC voltage obtained by the smoothing. In this case, the phase difference becomes smaller as the magnitude of the DC voltage becomes larger, and conversely, the phase difference becomes larger as the magnitude of the DC voltage becomes smaller.

As described below, the control unit 3112 controls the excitation-signal oscillator portion 3102 (the oscillation frequency f). In addition, the temperature information is input to the control unit 3112 from the temperature sensor 3110.

[Bode diagram]

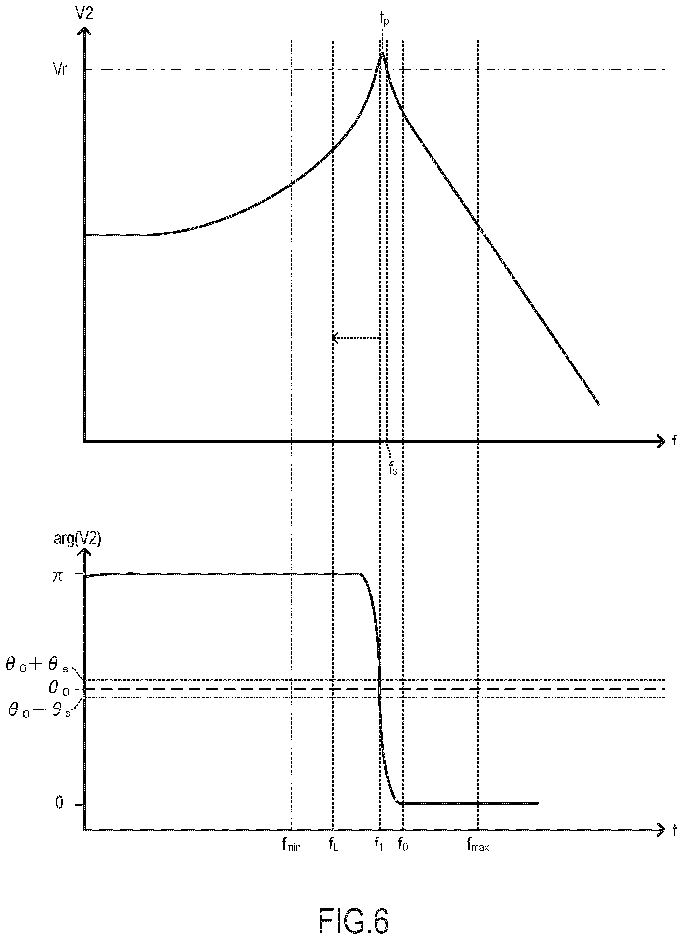

FIG. 6 is a diagram for explaining the gain and the phase difference $\theta$ detected by the fluid-property detection device 1 according to this embodiment. FIG. 6 is a Bode diagram of the circuit including the resonant circuit 8 and the capacitor C2, with the upper graph representing the gain and the lower graph representing the phase difference $\theta$. In this embodiment, the gain is obtained by calculating (V2/V1) using the voltage V1 detected by the first voltage sensor 3107 shown in FIG. 4 and the voltage V2 detected by the second voltage sensor 3108. In addition, the phase difference is represented by arg (V2/V1).

Because the voltage V1 (amplitude, phase) of the excitation signal is known, the gain and the phase difference $\theta$ may be calculated only from V2. Thus, in FIG. 6, the voltage (V2) is applied as the gain, and arg (V2) is applied as the phase difference $\theta$.

As shown in FIG. 6, in the circuit including the resonant circuit 8 and the capacitor C2, the phase difference $\theta$ exhibits characteristics where it changes significantly before and after the resonance frequency f0, which is formed by the capacitor C1 and the equivalent inductance L, and the resonance frequency f1, which is formed by the inductance L and the combined capacitance of the capacitor C1 and the capacitor C2. Because the capacitor C2 is sufficiently small compared with a capacitor C1, the difference between the resonance frequency f0 and the resonance frequency f1 is small as described above (in FIG. 6, in order to secure viewability, the resonance frequency f0 and the resonance frequency f1 are shown slightly separated from each other).

Only within a narrow frequency range in the vicinity of the resonance frequency f0 and the resonance frequency f1, the amplitude and the phase of the voltage applied to the excitation electrode 28 can be considered constant. Therefore, as shown in FIG. 6, when viewed from the high-frequency side, the phase difference $\theta$ remains 0 up to the resonance frequency f0, but rapidly rises towards the lower frequency side and becomes $\theta0$

(for example, $\pi/2$) at the resonance frequency f1 and becomes $\pi$ at even lower frequency.

The frequency fp at which the gain (the voltage V2) peaks is located between the resonance frequency f1 and the resonance frequency f0 and adjacent to the resonance frequency f1.

In the present invention, as described below, in a state in which the property of the working oil (fluid) is normal, the phase difference $\theta$ between the excitation signal and the output signal from the resonant circuit 8 is detected at the frequency at which the slope of the curve indicating the phase difference $\theta$ shown in FIG. 6 becomes the largest.

When the property of the working oil, which is filled between the gaps formed by the detection electrode 21 with the inner electrode 22 and the cover electrode 27, is changed and the electrical parameter (the relative permittivity) of the working oil is changed, the magnitude of the capacitor C1 is changed and the resonance frequency f0 and the resonance frequency f1 are changed. In general, when oil, etc. is deteriorated, the relative permittivity thereof is increased, and thereby, the resonance frequency f0 and the resonance frequency f1 are shifted towards the low frequency side. Even if this shift amount is small, because the slope of the curve indicating the phase difference $\theta$ shown in FIG. 6 is very steep, even a small change in the relative permittivity can be sensitively detected as the phase difference $\theta$ under a state in which the oscillation frequency f of the excitation signal is constant.

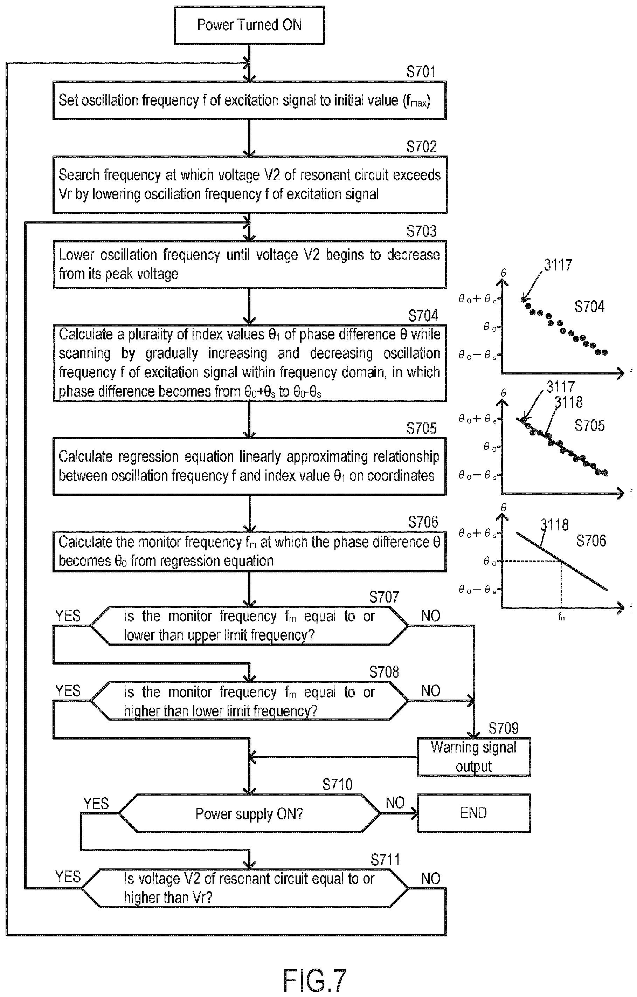

In addition, in the present invention, as described below, the oscillation frequency f is searched within the frequency domain in the vicinity of the resonance frequency f1 at which the phase difference $\theta$ can be sensitively detected, and a monitor frequency $f_m$ at which the phase difference $\theta$ becomes a predetermined phase difference ($\theta$0) is calculated. Then, the monitor frequency $f_m$ is set through feedback control (see FIG. 7) such that the phase difference $\theta$ is maintained at a predetermined phase difference ($\theta$0). Therefore, in the present invention, it is possible to sensitively detect the property of the working oil through the change in the monitor frequency $f_m$. In this case, the oscillation frequency f is constantly changed in a manner that it reciprocates around the resonance frequency f1.

Note that, in the present invention, for example, it is possible to estimate the absolute value of the electrical parameter (the relative permittivity) of the working oil by using the oscillation frequency f (the resonance frequency f1) at which the phase difference $\theta$ becomes n/2 by searching for the oscillation frequency f together with the capacitor C1 and the capacitor C2 (assumed to be known). Similarly, it is also possible to estimate the absolute value of the electrical parameter (the relative permittivity) of the working oil by using the oscillation frequency f (the resonance frequency f0) at which the phase difference $\theta$ becomes 0 by searching for the oscillation frequency f together with the capacitor C1 (assumed to be known).

sumed to be known).

In conventional technique (see Patent Literature 1), when expressed in terms of the present embodiment, the excitation electrode 28 to which the excitation signal is applied is directly connected to the detection electrode 21, the capacitor C2 shown in FIG. 4 is not provided, and an internal resistance component (output impedance) of the element that outputs the excitation signal is directly connected to the resonant circuit 8. In this case, the internal resistance component is included in the Q value that is determined by the ratio of the magnitude of the inductance component to the magnitude of the resistance component of the circuit including the resonant circuit 8, and therefore, it becomes difficult to increase the Q value. Thus, the peak of the gain shown in FIG. 6 cannot be made steep, and the change in the phase difference $\theta$ also becomes gradual. Consequently, the detection sensitivity for the phase difference is decreased, and it is difficult to perform monitoring for sensitively detecting the change in the property of the working oil.

On the other hand, in this embodiment, the capacitor C2 (the parasitic capacitance) is interposed between the internal resistance component (the output impedance) of the excitation-signal oscillator portion 3102 and the resonant circuit 8, and so, the internal resistance component of the excitation-signal oscillator portion 3102 is separated away from the resonant circuit 8. In addition, in the inductor portion 3101, as described above, it is possible to set the inductance L to a large value by setting C4, R1, and R5 to large resistance values. Because the Q value in the resonant circuit 8 is determined by the ratio of the impedance of the inductance L to the resistance component that equivalently exists in the inductor portion 3101, it is possible to set the Q value to a very large value. Therefore, the peak of the gain shown in FIG. 6 can be made steep, and in addition, the change in the phase difference $\theta$ also becomes steep. Consequently, the detection sensitivity for the phase difference is improved, and it becomes possible to perform the monitoring for sensitively detecting the change in the property of the working oil.

[Control in First Embodiment]

FIG. 7 is a control flow of the fluid-property detection device 1 according to the first embodiment.

In step S701, when the device power is turned ON, the control unit 3112 initially sets the oscillation frequency f of the excitation signal to a frequency $f_{max}$ having higher frequency than the resonance frequency f0 (a first control).

In step S702, the control unit 3112 searches for the oscillation frequency f (located in the vicinity of the resonance frequencies f0 and f1) at which the amplitude (the voltage V2) of the output signal from the resonant circuit 8 becomes equal to or higher than a predetermined threshold voltage (Vr) (the first control).

In the first control, as shown in FIG. 6, the control unit 3112 initially sets the oscillation frequency f of the excitation signal to the frequency $f_{max}$ having higher frequency than the resonance frequency f0, lowers the oscillation frequency f, and stops the search (scan) of the oscillation frequency f as the first control when the voltage V2 reaches Vr (the oscillation frequency f is fs shown in FIG. 6). As described above, because the search is performed for the frequency domain in which the voltage V2 reaches near the peak from the high-frequency side, even if there is a high-frequency component in the excitation signal, it is possible to avoid search failure caused by the resonant circuit 8 responding to the high-frequency component and causing the voltage V2 to exceed Vr. Note that, when there is no influence of the high-frequency component, it may be possible to perform a control in which the frequency is increased from a frequency $f_{min}$, which is lower than both of the resonance frequency f0 and the resonance frequency f1, and the frequency is continuously increased until the voltage V2 reaches Vr.

In step S703, the control unit 3112 lowers the search speed compared with the first control and continues to lower the oscillation frequency f until the voltage V2 reaches the peak voltage and begins to decrease (a second control). In other words, the control unit 3112 lowers the oscillation frequency f to the frequency in the vicinity of the frequency fp, at which the voltage V2 peaks, and lower than the frequency fp (see FIG. 6). In this case, for example, the difference between the current data and the previous data of the voltage V2 is calculated, and when the positive/negative sign of the differences is inverted, the process of step S703 is terminated.

In step S704, the control unit 3112 calculates the index value θ1 of the phase difference θ while searching for the oscillation frequency f at a low speed within the frequency domain, in which the phase difference θ becomes from $\theta_0-\theta_s$ to $\theta_0+\theta_s$, thereby acquiring a large number of dot data 3117 indicating the coordinate positions of the oscillation frequency f and the index value $\theta_1$ in the coordinate space having the oscillation frequency f and the phase difference θ as the coordinate axes (the second control). Note that, $\theta_s$ is an arbitrary value at which the first control can function. In addition, the control unit 3112 has a memory (not shown) for storing the dot data 3117.

In step S705, using the coordinate space, the control unit 3112 calculates the regression equation that expresses the correlation between the oscillation frequency f and the phase difference θ as the linear function 3118 from the large number of the dot data 3117 (the second control). Here, the regression equation is θ = A·f+B, and the values A and B in the regression equation are calculated. In addition, after calculating the regression equation, the control unit 3112 deletes the dot data 3117 stored in the memory (not shown).

In step S706, using the regression equation, the control unit 3112 calculates the monitor frequency $f_m$ at which the phase difference θ becomes $\theta_0$ (for example π/2) (the second control).

In step S707, the control unit 3112 determines whether or not the calculated monitor frequency $f_m$ is equal to or less than the upper limit frequency $f_H$, and when it is determined as YES, the process proceeds to step S708, and when it is determined as NO, the process proceeds to step S709.

In step S708, the control unit 3112 determines whether or not the monitor frequency $f_m$ is equal to or higher than the lower limit frequency $f_L$, and when it is determined as YES, the process proceeds to step S710, and when it is determined as NO, the process proceeds to step S709.

In step S709, the control unit 3112 outputs an warning signal to the external system. Here, when the process has proceeded from step S707 to step S709, the control unit 3112 outputs the upper limit warning signal to the external system. When the working oil is contaminated with other types of oil or air bubbles, the capacitor $C_1$ is decreased, causing the resonance frequencies $f_0$ and $f_1$ to be increased, making it difficult to accurately measure changes in the electrical parameter (the relative permittivity) of the working oil. Thus, in the present invention, when the monitor frequency $f_m$ (the oscillation frequency f) exceeds the upper limit frequency $f_H$, it is determined that the working oil is contaminated with other types of oil or air bubbles, and a report is sent to the external system.

The upper limit warning signal (and the lower limit warning signal, which will be described later) is transmitted to an operator by being converted into, for example, an audio output from a speaker, a lighting display of a warning lamp, a warning display on an image display device, or the like. By doing so, the operator can be notified of the replacement of the working oil.

When the process has proceeded from step S708 to step S709, the control unit 3112 outputs the lower limit warning signal to the external system. The relative permittivity of the working oil is increased due to oxidation of the working oil, mixing of contaminants (metal pieces) formed by wear of the hydraulic device (not shown), on which the pipe 7 is provided, into the working oil, or the like. The increase in the relative permittivity of the working oil results in the increase in the capacitance of the capacitor $C_1$, and thereby, the monitor frequency $f_m$ that is calculated by the second control under a condition in which the first control is functioning is lowered. The change in the monitor frequency $f_m$ appears as the change in the electrical parameter (the relative permittivity) of the working oil.

Thus, in the present invention, when the calculated monitor frequency $f_m$ (the oscillation frequency f) becomes lower than the lower limit frequency $f_L$, it is determined that the time has come to replace the working oil, and a report is sent to the external system. Although the lower limit warning signal is similar to the upper limit warning signal, it is preferable that it be in a form that allows the operator to distinguish between the two.

In step S710, the control unit 3112 determines whether or not the device power is ON, and when it is determined as YES, the process proceeds to step S711, and when it is determined as NO, the control is terminated.

In step S711, the control unit 3112 determines whether or not the voltage V2 is equal to or higher than Vr, and when it is determined as YES, the process proceeds to step S703, and when it is determined as NO, the process proceeds to step S701. By making determination as NO in step S711, it is possible to recover from a state in which the second control cannot be executed.

As shown in FIG. 5, although the inductor portion 3101 has the resistors R1, R2, R3, and R5, they are subjected to resistance value change by change in the temperature. In addition, because the operational amplifier $U_1$ and the operational amplifier $U_2$ are semiconductor devices, their operating characteristics are affected by temperature. Therefore, the inductance L is changed by the temperature change in the inductor portion 3101. In addition, the relative permittivity of the working oil is changed with temperature, and thereby, the capacitor $C_1$ (the capacitance) is changed.

Thus, the control unit 3112 has a map in which the temperature of the inner electrode 22 (the temperature of the working oil) (or the temperature of the inductor portion 3101) is correlated with the amount of change (correction amount) in the monitor frequency $f_m$ (the oscillation frequency f) of the excitation signal under the condition in which the first control is functioning. Then, on the basis of the temperature information of the inductor portion 3101 that is input from the temperature sensor 3110, the control unit 3112 extracts the amount of change (the correction amount) in the resonance frequency f0 (or the resonance frequency f1) from the map, and uses the amount of change (the correction amount) to correct (temperature correction) the information of the oscillation frequency f of the excitation signal under the condition in which the first control is functioning (a third control). By doing so, it is possible to correct the temperature error of the information of the monitor frequency $f_m$ (the oscillation frequency f) to be calculated.

The control unit 3112 may execute a control in which the information of the monitor frequency $f_m$ (the oscillation frequency f), or the information of the difference between the monitor frequency $f_m$ (the oscillation frequency f) and the lower limit frequency $f_L$ is output to the external system. By doing so, the operator can set the threshold value in accordance with the oil type of the working oil, the usage conditions of equipment, and so forth, and so, it is possible to optimize the timing for replacing the working oil used in the equipment.

[Second Embodiment]

FIG. 8 is a circuit diagram of the fluid-property detection device 1 according to the second embodiment.

In the fluid-property detection device 1 of the second embodiment, the excitation signal is the square wave. In addition, the control unit 3112 is formed by a microcomputer and can output the excitation signal. The excitation signal is a digital square wave (rectangular wave) with a duty ratio of 50% and its oscillation frequency f is controlled by the microcomputer.

The control unit 3112 outputs the excitation signal to the phase comparator 3105 and the excitation electrode 28 by using a counter of the microcomputer or a built-in oscillation module. The first low-pass filter 3115 is arranged between the control unit 3112 and the excitation electrode 28, and the excitation signal that has passed the first low-pass filter 3115 is output to the excitation electrode 28.

The first low-pass filter 3115 is a low-pass filter whose cutoff frequency is set to about the resonance frequency f0.

Although the inductor portion 3101 has a similar configuration as that in the first embodiment, the output terminal extends from between the capacitor C4 and the resistor R5.

The output from the inductor portion 3101 is output to a third comparator 3113 and a rectifier circuit 3114 via a buffer U3.

The third comparator 3113 shapes the output from the inductor portion 3101 (the output signal from the resonant circuit 8) into the digital square wave and outputs it to the phase comparator 3105. The output signal from the third comparator 3113 becomes a signal that reflects the phase of the output signal from the resonant circuit 8.

The rectifier circuit 3114 rectifies the output from the inductor portion 3101 (the output signal from the resonant circuit 8) into direct current and outputs it to a second low-pass filter 3116.

The second low-pass filter 3116 smoothens the output of the rectifier circuit 3114 and outputs it to the control unit 3112. The output of the second low-pass filter 3116 serves as an indicator of the amplitude of the output signal from the resonant circuit 8 (corresponding to the voltage V2 of the first embodiment).

The phase comparator 3105 measures the timing of the rising edge (or the falling edge) of the excitation signal output from the control unit 3112 and the timing of the rising edge (or the falling edge) of the output signal from the third comparator 3113 (the output signal from the resonant circuit 8) and outputs, on the basis of the time difference therebetween, to the control unit 3112 the square wave (the third square wave in the first embodiment) representing the phase difference θ between the excitation signal and the output signal from the resonant circuit 8.

The phase comparator 3105 may be configured by using an exclusive OR circuit (XOR gate). In this case, one input is the excitation signal, and the other input is the output signal from the third comparator 3113, and the output becomes a square wave corresponding to the time difference between the rising edges of the one input and the other input and the time difference between the falling edges of the one input and the other input.

In addition, the phase comparator 3105 may be configured by using a D flip-flop. In this case, for example, if the D flip-flop corresponding to 74LS74 is to be used, by inputting the excitation signal to a CK terminal, by inputting the output signal from the third comparator 3113 (the signal with inverted voltage) to a D terminal, and by inputting the output signal from the third comparator 3113 to a CLR-terminal, it is possible to output, from a Q terminal, the square wave corresponding to the phase delay of the output signal from the third comparator 3113 with respect to the excitation signal. However, the maximum value of the detectable phase difference θ is 180° (π).

Furthermore, the phase comparator 3105 may be configured by using two D flip-flops. In this case, for example, by inputting the excitation signal to the CK terminal of a first D flip-flop, by setting the input of the D terminal of the first D flip-flop to H (high potential), by inputting the output signal from the third comparator 3113 to the CK terminal of a second D flip-flop, by connecting the D terminal of the second D flip-flop to the Q terminal of the first D flip-flop, and by inputting, to the CLR-terminal of the first flip-flop and the CLR-terminal of the second flip-flop, the output from a NAND gate having the output from the Q terminal of the first flip-flop and the output from the Q terminal of the second flip-flop as inputs, it is possible to output the square wave corresponding to the phase delay of the output signal from the third comparator 3113 with respect to the excitation signal from the Q-terminal of the first D flip-flop over the phase difference θ of 360° (2 π).

Note that, some of the microcomputers have a built-in function of measuring a time difference (the phase difference θ) between two signals. Thus, when the microcomputer to be used for the control unit 3112 has such a function, the phase comparator 3105 may be omitted.

The control flow executed by the control unit 3112 is similar to that in the first embodiment.

In step S702 mentioned above, the control unit 3112 searches for the oscillation frequency f (located near the resonance frequencies f0 and f1) at which the output voltage from the second low-pass filter 3116 becomes equal to or higher than a predetermined threshold voltage (the voltage corresponding to the threshold voltage (Vr) in the first embodiment) (the first control).

In step S703 mentioned above, the control unit 3112 lowers the search speed compared with that in the first control and lowers the oscillation frequency f until the output voltage from the second low-pass filter 3116 reaches the peak voltage and begins to decrease (the second control).

Because the inductor portion 3101 (the GIC circuit) is the circuit excited through the capacitor C2, the overall electrical signals are small and susceptible to noise. In particular, not only in the inductor portion 3101 but also in various portions of the circuit, the signal itself flowing therethrough becomes a signal with the same frequency as the excitation signal, and so, the signal may cause the resonant circuit 8 to resonate through the parasitic capacitances present in various portions of the circuit, and there is a risk in that the circuit operation becomes unstable.

Therefore, as shown in the rectangular region enclosed by the dash-dot line in FIG. 8, it is preferable to divide the resistor R5 into R6 and R7, and to configure the output terminal to extend from between R6 and R7. In this case, for example, R5 = R 6 + R7, and it is preferable to set R7 to approximately 1/10 of the resistance value of R6. By doing so, the output voltage of the inductor portion 3101 is divided to a voltage of 1/10 or less, and so, it is possible to suppress unnecessary resonance, in other words, the resonance of the resonant circuit 8 due to the output signal output from the inductor portion 3101.

Although the illustration is omitted, the resistor R7 actually consists of two resistance element, each having twice the resistance value of the resistor R7, connected in parallel to the resistor R6. One of the resistance elements is grounded, and the other of the resistance elements is connected to a positive power supply line (for example, 5V) (not shown). Therefore, the other of the resistance elements is grounded with respect to the AC signal (the excitation signal) input from the external system of the inductor portion 3101, and a DC bias of half of the power supply voltage is applied. This enables operation with a 5V DC power supply, for example.

Note that, the operational advantages achieved in the second embodiment are the same as those in the first embodiment.

[Third Embodiment]

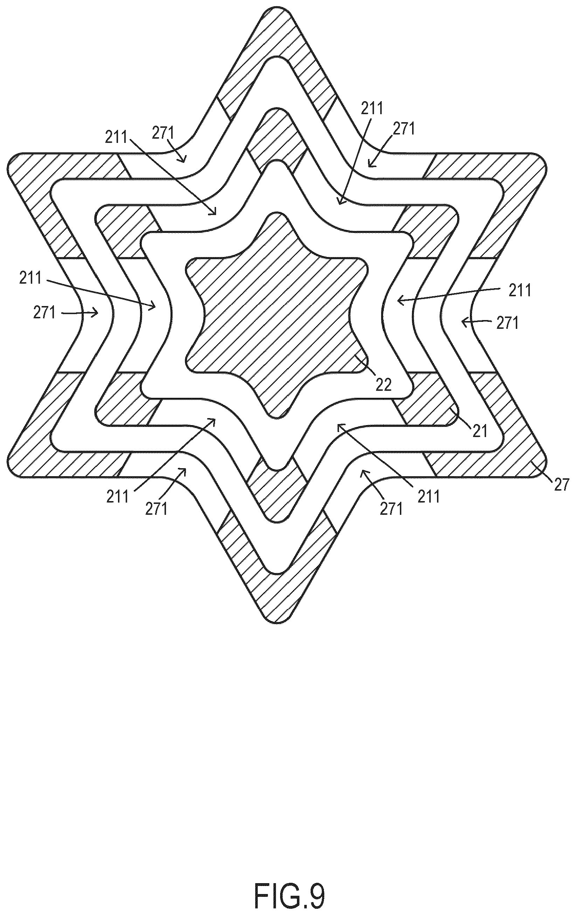

FIG. 9 is a sectional view of the inner electrode 22, the detection electrode 21, and the cover electrode 27 forming the fluid-property detection device 1 according to a third embodiment. As shown in FIG. 9, the inner electrode 22, the detection electrode 21, and the cover electrode 27 in this modification each has a star-shaped (hexagram) cross-sectional shape in the radial direction. In the cover electrode 27, for example, the flow holes 271 are formed in a star-shaped saddle portion, and similarly, the flow holes 211 are formed in the detection electrode 21. With the above-described configuration, the facing surface area between the detection electrode 21 and the inner electrode 22 and the facing surface area between the detection electrode 21 and the cover electrode 27 are increased, and so, the capacitance of the capacitor C1 can be correspondingly increased, and the resonance frequency f0 shown in the equation (1) and the resonance frequency f1 shown in the equation (2) can be set to lower values.

[Configurations, Operations, and Effects of Present Embodiments]

In the following, the configurations, operations, and effects of the present embodiments (the first to third embodiments) will be collectively described.

The fluid-property detection device 1 according to this embodiment includes: the resonant circuit 8 including the ground electrode (the inner electrode 22, the cover electrode 27) configured to be immersed in the fluid, the detection electrode 21 configured to be immersed in the fluid and arranged so as to face the ground electrode (the inner electrode 22, the cover electrode 27), and the inductor portion 3101 connected to the detection electrode 21, the resonant circuit 8 being formed by electrically connecting the capacitor C1 and the inductor portion 3101 to each other, the capacitor C1 being formed by the ground electrode (the inner electrode 22, the cover electrode 27) and the detection electrode 21; the excitation-signal oscillator portion 3102 configured to excite the resonant circuit 8 with the excitation signal having a predetermined oscillation frequency f; a phase difference detector (the first comparator 3103, the second comparator 3104, the phase comparator 3105) configured to detect the phase difference $\theta$ between the excitation signal and the output signal from the resonant circuit 8; and the control unit 3112 configured to control the oscillation frequency f such that the phase difference $\theta$ maintains a predetermined phase difference ($\theta 0$), wherein it is characterized in that the fluid-property detection device 1 detects the fluid property through the change in the oscillation frequency f.

According to the above-described configuration, because the oscillation frequency f can always be controlled so as to be the frequency at which the phase change in the resonant circuit 8 is the steepest, even if the capacitance of the capacitor C1, in other words, the relative permittivity of the fluid is changed, the oscillation frequency f can follow the frequency, and so, the maximum sensitivity to the phase change can be maintained at all times. Therefore, by improving the detection sensitivity for the phase difference between the excitation signal and the output signal from the resonant circuit 8, it becomes possible to perform the monitoring for sensitively detecting the change (temporal deterioration) in the fluid property.

In the fluid-property detection device 1 of this embodiment, the fluid-property detection device 1 is further provided with the excitation electrode 28 connected to the excitation-signal oscillator portion 3102, the excitation electrode 28 being arranged in the vicinity of the detection electrode 21 in a state where the excitation electrode 28 is separated away from the detection electrode 21, wherein it is characterized in that the excitation-signal oscillator portion 3102 excites the resonant circuit 8 by outputting the excitation signal to the resonant circuit 8 via the parasitic capacitance (the capacitor C2) formed between the excitation electrode 28 and the detection electrode 21.

According to the above-described configuration, because the excitation electrode 28 to which the excitation signal is applied is spatially separated from the detection electrode 21, there is no DC resistance component between the excitation electrode 28 and the detection electrode 21, and both are electrically connected via the parasitic capacitance (the capacitor C2), and so, it is possible to improve the Q value of the resonant circuit 8. Thus, by improving the detection sensitivity for the phase difference between the excitation signal and the output signal from the resonant circuit 8, it becomes possible to perform the monitoring for sensitively detecting the change (deterioration over time) in the fluid property.

In the fluid-property detection device 1 of this embodiment, it is characterized in that the inductor portion 3101 is the equivalent inductor including a plurality of impedance components each does not include an inductor.

According to the above-described configuration, because the inductance L can be set sufficiently larger than that of a normal inductor element, the resonance frequencies f0 and the resonance frequency f1 can be set to lower values, and thereby, it is possible to set the oscillation frequency f to a lower value. Therefore, it is possible to construct, in addition to the inductor portion 3101, the excitation-signal oscillator portion 3102 and the phase difference detectors (the first comparator 3103, the second comparator 3104, and the phase comparator 3105) by combining general-purpose electronic components (semiconductor devices), making it possible to achieve a low-cost and compact configuration.

In the fluid-property detection device 1 of this embodiment, it is characterized in that the impedance component includes a first resistor (R1) connected to the detection electrode 21, a second resistor (R2), a third resistor (R3), a second capacitor (C4), and a fourth resistor (R5) connected to the ground electrode (the inner electrode 22, the cover electrode 27), and the impedance component forms the series circuit in which the first resistor (R1), the second resistor (R2), the third resistor (R3), the second capacitor (C4), and the fourth resistor (R5) are connected in series in this order.

According to the above-described configuration, because the equivalent inductor is constructed by a so-called GIC circuit, a large inductance value is obtained.

In the fluid-property detection device 1 of this embodiment, it is characterized in that a fourth resistor (R5) includes a fifth resistor (R6) connected to the second capacitor (C4) and a sixth resistor (R7) connected to the ground electrode (the inner electrode 22, the cover electrode 27) and connected to the fifth resistor (R6), a resistance value of the sixth resistor (R7) is set so as to be lower than a resistance value of the fifth resistor (R6), and the output terminal of the resonant circuit 8 extends between the fifth resistor (R6) and the sixth resistor (R7).

According to the above-described configuration, because the output voltage from the resonant circuit 8 (the inductor portion 3101) can be made smaller, it is possible to suppress the oscillation of the resonant circuit 8 (the inductor portion 3101) by the output voltage.

In the fluid-property detection device 1 of this embodiment, it is characterized in that the control unit 3112 sets the oscillation frequency f to the resonance frequency of the resonant circuit 8 (the resonance frequency f0, the resonance frequency f1) or to the frequency in the vicinity of the resonance frequency (the resonance frequency f0, the resonance frequency f1).

According to the above-described configuration, by at least setting the oscillation frequency f to the frequency in the vicinity of the resonance frequency (the resonance frequency f0, the resonance frequency f1), the phase difference θ between the excitation signal (the voltage) and the output signal (the voltage) of the resonant circuit 8 is changed sensitively in response to the change in the state of the fluid, and therefore, it is possible to improve the detection sensitivity for the phase difference.

In the fluid-property detection device 1 of this embodiment, the fluid-property detection device 1 further includes an amplitude detector (the second voltage sensor 3108, the second low-pass filter 3116, the control unit 3112) configured to detect the amplitude of the output signal, wherein it is characterized in that the control unit 3112 searches and detects the frequency domain in which the amplitude (the voltage V2) becomes equal to or higher than a predetermined threshold value (Vr) by changing the oscillation frequency f, calculate the correlation (the dot data 3117, the linear function 3118) between the oscillation frequency f and the phase difference θ by detecting the phase difference θ between the excitation signal and output signal while changing the oscillation frequency f in the frequency domain, and calculate the monitor frequency $f_m$ at which the phase difference θ becomes a predetermined phase difference (θ0) based on the correlation (the dot data 3117, the linear function 3118).

According to the above-described configuration, for the phase difference θ between the excitation signal and the output signal, because the monitor frequency $f_m$ (the oscillation frequency f) can be calculated with high accuracy within the phase difference θ range corresponding to the frequency at which the phase change in the resonant circuit 8 is the steepest, even if the capacitance of the capacitor C1, in other words, the relative permittivity of the fluid is changed, the monitor frequency $f_m$ (the oscillation frequency f) can follow the frequency with high accuracy, and so, the maximum sensitivity to the phase change can be maintained at all times.

In the fluid-property detection device 1 of this embodiment, it is characterized in that, when the frequency domain is to be searched, the control unit 3112 searches the frequency domain by decreasing the oscillation frequency f from the frequency higher than the frequency domain.

When the excitation signal includes the harmonic component and when the harmonic component is close to the resonance frequency of the resonant circuit 8 (the resonance frequency f0, the resonance frequency f1), there is a risk in that the resonant circuit 8 responds to the harmonic component and causes a problem in the search for the frequency domain. However, with the above-described configuration, because the frequency domain is searched by decreasing the oscillation frequency from the frequency that is higher than the frequency domain, the resonant circuit 8 does not respond to the harmonic component, and so, it is possible to reliably perform the search for the frequency domain.

In the fluid-property detection device 1 of this embodiment, the fluid-property detection device 1 further includes the temperature sensor 3110 configured to measure the temperature of the inductor portion 3101 or the temperature of the fluid, wherein it is characterized in that the control unit 3112 calculates the correction amount of the resonance frequency (the resonance frequency f0, the resonance frequency f1) of the resonant circuit 8 based on the temperature detected by the temperature sensor 3110.

According to the above-described configuration, by correcting the information of the oscillation frequency f or the information of the monitor frequency $f_m$ based on the calculated correction amount, it is possible to detect with high accuracy in a short time the property of the fluid (the working oil) through the information of the oscillation frequency f after correction or the information of the monitor frequency $f_m$ after correction regardless of the temperature change of the fluid (the working oil).

In the fluid-property detection device 1 of this embodiment, it is characterized in that, when the information of the oscillation frequency f or the information of the monitor frequency $f_m$ becomes lower than a predetermined lower limit frequency $f_L$ or when the information of the oscillation frequency f or the information of the monitor frequency $f_m$ exceeds a predetermined upper limit frequency $f_H$, the control unit 3112 outputs the warning signal to the external system.

According to the above-described configuration, by outputting the warning signal when the information of the oscillation frequency f or the information of the monitor frequency $f_m$ exceeds a predetermined upper limit frequency $f_H$, it is possible to notify the operator that the abnormality has occurred in the fluid, and by outputting the warning signal when the information of the oscillation frequency f or the information of the monitor frequency $f_m$ becomes lower than a predetermined lower limit frequency $f_L$, it is possible to notify the operator that the fluid should be replaced.

In the fluid-property detection device 1 of this embodiment, it is characterized in that the control unit 3112 outputs, to the external system, at least one of the information of the oscillation frequency f, the information of the difference between the oscillation frequency f and a predetermined lower limit frequency $f_L$, the information of the monitor frequency $f_m$, and the information of the difference between the monitor frequency $f_m$ and the lower limit frequency fL.

According to the above-described configuration, by outputting, to the external system, at least one of the information of the oscillation frequency f, the information of the difference between the oscillation frequency f and a predetermined lower limit frequency $f_L$, the information of the monitor frequency $f_m$, and the information of the difference between the monitor frequency $f_m$ and the lower limit frequency fL, the operator can set the threshold value in accordance with the type of fluid, the usage conditions of the equipment, and so forth, and so, it is possible to optimize the timing for replacing the fluid used in the equipment.

In the fluid-property detection device 1 of this embodiment, the fluid-property detection device 1 further includes the amplitude detector (the second voltage sensor 3108, the second low-pass filter 3116, the control unit 3112) configured to detect the amplitude of the output signal, wherein it is characterized in that, when the amplitude (the voltage V2) becomes lower than a predetermined threshold value (Vr), the control unit 3112 searches and detects the frequency domain in which the amplitude (the voltage V2) becomes equal to or higher than the threshold value (Vr) by changing the oscillation frequency f and control the oscillation frequency f within the frequency domain.

According to the above-described configuration, when, for example, the fluid property or the property of the electrode is changed in a short time and the resonance frequency (the resonance frequency f0, the resonance frequency f1) is changed rapidly, the output signal is changed, and a situation in which the phase difference θ between the excitation signal and the output signal from the resonant circuit 8 can no longer maintain a predetermined phase difference (θ0) is caused. Thus, by searching and detecting the frequency domain in which the output signal becomes equal to or higher than a predetermined amplitude (Vr) and by controlling the oscillation frequency f within this frequency domain, it is possible to cause the oscillation frequency f to follow the resonance frequency (the resonance frequency f0, the resonance frequency f1) again such that the phase difference θ between the excitation signal and the output signal from the resonant circuit 8 becomes a predetermined phase difference (θ0).

In the fluid-property detection device 1 of this embodiment (the second embodiment), it is characterized in that, the fluid-property detection device 1 further includes the low-pass filter (the first low-pass filter 3115) arranged between the excitation-signal oscillator portion 3102 and the excitation electrode 28.

When the excitation signal includes the harmonic component and when the harmonic component is close to the resonance frequency of the resonant circuit 8 (the resonance frequency f0, the resonance frequency f1), there is a risk in that the resonant circuit 8 responds to the harmonic component and causes a problem in the search for the frequency domain. However, with the above-described configuration, because input of the harmonic component of the excitation signal to the resonant circuit 8 is limited, the response of the resonant circuit 8 to the harmonic component is suppressed, and it is possible to reliably perform the search for the frequency domain.

In the fluid-property detection device 1 of this embodiment (the first embodiment), it is characterized in that, when the excitation signal output by the excitation-signal oscillator portion 3102 is a sinusoidal wave, the phase difference detector (the first comparator 3103, the second comparator 3104, the phase comparator 3105) includes: the first square wave generator (the first comparator 3103) that outputs the first square wave on the basis of one of the positive sign and the negative sign of the excitation signal; the second square wave generator (the second comparator 3104) that outputs the second square wave on the basis of the one of the positive sign and the negative sign of the output signal; and the phase comparator 3105 that detects the phase difference θ by acquiring the information of the time difference between the rising edges or the falling edges of the first square wave and the second square wave.

According to the above-described configuration, it is possible to detect, with a simple configuration, the phase difference θ between the excitation signal and the output signal from the resonant circuit 8.

In the fluid-property detection device 1 of this embodiment (the second embodiment), it is characterized in that, when the excitation signal oscillated by the excitation-signal oscillator portion 3102 is the square wave, the phase difference detector (the phase comparator 3105) detects the phase difference θ by acquiring the information of the time difference between the rising edges or the falling edges of the excitation signal and output signal.

According to the above-described configuration, it is possible to detect, with a simple configuration, the phase difference θ between the excitation signal and the output signal from the resonant circuit 8.

In the fluid-property detection device 1 of this embodiment (the third embodiment), it is characterized in that the surface of the ground electrode (the inner electrode 22, the cover electrode 27) facing the detection electrode 21, and the surface of the detection electrode 21 facing the ground electrode (the inner electrode 22, the cover electrode 27) each has a wave shape.

According to the above-described configuration, by increasing the facing surface areas of the ground electrode (the inner electrode 22, the cover electrode 27) and the detection electrode 21, the proportion of effective capacitance in the capacitor C1 that is formed by the ground electrode (the inner electrode 22, the cover electrode 27) and the detection electrode 21 can be increased, and so, it is possible to improve the detection sensitivity.

The fluid-property detection method according to this embodiment includes: the ground electrode (the inner electrode 22, the cover electrode 27) at least partially immersed in the fluid; the detection electrode 21 at least partially immersed in the fluid and arranged so as to face the ground electrode (the inner electrode 22, the cover electrode 27); and the inductor portion 3101 connected to the detection electrode 21, wherein the method is characterized by comprising a step of exciting, by the excitation signal having a predetermined oscillation frequency f, the resonant circuit 8 that is formed by electrically connecting the inductor portion 3101 and the capacitor C1, which is formed by the ground electrode (the inner electrode 22, the cover electrode 27) and the detection electrode 21, a step of detecting the phase difference $\theta$ between the excitation signal and the output signal from the resonant circuit 8, and a step controlling the oscillation frequency f such that the phase difference $\theta$ maintains a predetermined phase difference ($\theta$0), thereby detecting the fluid property through the change in the oscillation frequency f.

According to the above-described method, because the oscillation frequency f can always be controlled so as to be the frequency at which the phase change in the resonant circuit 8 is the steepest, even if the capacitance of the capacitor C1, in other words, the relative permittivity of the fluid is changed, the oscillation frequency f can be caused to follow the frequency, and the maximum sensitivity to the phase change can be maintained at all times. Therefore, by improving the detection sensitivity for the phase difference between the excitation signal and the output signal from the resonant circuit 8, it becomes possible to perform the monitoring for sensitively detecting the change (deterioration over time) in the fluid property.

In the fluid-property detection method of this embodiment, it is characterized in that the resonant circuit 8 is excited by applying the excitation signal to the excitation electrode 28 that is arranged in the vicinity of the detection electrode 21 in a state in which the excitation electrode 28 is separated away from the detection electrode 21, and by outputting the excitation signal to the resonant circuit 8 via the parasitic capacitance (the capacitor C2) that is formed between the excitation electrode 28 and the detection electrode 21.

According to the above-described method, because the excitation electrode 28 to which the excitation signal is applied and the detection electrode 21 are spatially separated, there is no resistance component between the excitation electrode 28 and the detection electrode 21, and both are electrically connected via the parasitic capacitance (the capacitor C2), and so, it is possible to improve the Q value of the resonant circuit 8. Thereby, the detection sensitivity for the phase difference between the excitation signal and the output signal from the resonant circuit 8 is improved, and it becomes possible to perform the monitoring for sensitively detecting the change in the fluid property.

In the fluid-property detection method of this embodiment, it is characterized in that the method includes a step of searching and detecting the frequency domain in which the amplitude of the output signal (the voltage V2) exceeds a predetermined threshold value (Vr), a step of calculating the correlation (the dot data 3117, the linear function 3118) between the oscillation frequency f and the phase difference $\theta$ by detecting the phase difference $\theta$ between the excitation signal and the output signal while changing the oscillation frequency f within the frequency domain, and a step of calculating the monitor frequency $f_m$ at which the phase difference $\theta$ becomes a predetermined phase difference ($\theta$0) on the basis of the correlation (the dot data 3117, the linear function 3118).

According to the above-described method, because the monitor frequency $f_m$ (the oscillation frequency f) can always be controlled with high accuracy so as to be the frequency at which the phase change in the resonant circuit 8 is the steepest, even if the capacitance of the capacitor C1, in other words, the relative permittivity of the fluid is changed, the monitor frequency $f_m$ (the oscillation frequency f) can follow the frequency with high accuracy, and so, the maximum sensitivity to the phase change can be maintained at all times.

In the fluid-property detection method of this embodiment, it is characterized in that, when the frequency domain is searched, the frequency domain is searched by decreasing the oscillation frequency f from the frequency higher than the frequency domain.

When the excitation signal includes the harmonic component and when the harmonic component is close to the resonance frequency of the resonant circuit 8 (the resonance frequency f0, the resonance frequency f1), there is a risk in that the resonant circuit 8 responds to the harmonic component and causes a problem in the search for the frequency domain. However, with the above-described method, because the frequency domain is searched by decreasing the oscillation frequency from the frequency that is higher than the frequency domain, the resonant circuit 8 does not respond to the harmonic component, and so, it is possible to reliably perform the search for the frequency domain.

Embodiments of the present invention were described above, but the above embodiments are merely examples of applications of the present invention, and the technical scope of the present invention is not limited to the specific constitutions of the above embodiments.

This application claims priority based on Japanese Patent Application No. 2022-121000 filed with the Japan Patent Office on July 28, 2022, the entire contents of which are incorporated into this specification by reference.

**Claims**

1. A fluid-property detection device comprising: a resonant circuit including a ground electrode configured to be immersed in a fluid, a detection electrode configured to be immersed in the fluid and arranged so as to face the ground electrode, and an inductor portion connected to the detection electrode, the resonant circuit being formed by electrically connecting a capacitor and the inductor portion to each other, the capacitor being formed by the ground electrode and the detection electrode;

   an excitation-signal oscillator portion configured to excite the resonant circuit with an excitation signal having a predetermined oscillation frequency;
   a phase difference detector configured to detect a phase difference between the excitation signal and an output signal from the resonant circuit; and
   a control unit configured to control the oscillation frequency such that the phase difference maintains a predetermined phase difference, wherein the fluid-property detection device detects the fluid property through change in the oscillation frequency.

2. The fluid-property detection device according to Claim 1 further comprising an excitation electrode connected to the excitation-signal oscillator portion, the excitation electrode being arranged in a vicinity of the detection electrode in a state where the excitation electrode is separated away from the detection electrode, wherein
   the excitation-signal oscillator portion is configured to excite the resonant circuit by outputting the excitation signal to the resonant circuit via a parasitic capacitance formed between the excitation electrode and the detection electrode.

3. The fluid-property detection device according to Claim 1, wherein the inductor portion is an equivalent inductor comprising a plurality of impedance components each does not include an inductor.

4. The fluid-property detection device according to Claim 3, wherein the impedance component comprises a first resistor connected to the detection electrode, a second resistor, a third resistor, a second capacitor, and a fourth resistor connected to the ground electrode, and the impedance component forming a series circuit in which the first resistor, the second resistor, the third resistor, the second capacitor, and the fourth resistor are connected in series in this order.

5. The fluid-property detection device according to Claim 4, wherein the fourth resistor comprises a fifth resistor connected to the second capacitor and a sixth resistor connected to the ground electrode and connected to the fifth resistor,

   a resistance value of the sixth resistor is set so as to be lower than a resistance value of the fifth resistor, and
   an output terminal of the resonant circuit extends between the fifth resistor and the sixth resistor.

6. The fluid-property detection device according to Claim 1, wherein the control unit is configured to set the oscillation frequency to a resonance frequency of the resonant circuit or to a frequency in a vicinity of the resonance frequency.

7. The fluid-property detection device according to Claim 1, further comprising an amplitude detector configured to detect an amplitude of the output signal, wherein
   the control unit is configured to search and detect a frequency domain in which the amplitude becomes equal to or higher than a predetermined threshold value by changing the oscillation frequency, calculate a correlation between the oscillation frequency and the phase difference by detecting the phase difference between the excitation signal and the output signal while changing the oscillation frequency in the frequency domain, and calculate a monitor frequency at which the phase difference becomes the a predetermined phase difference based on the correlation.

8. The fluid-property detection device according to Claim 7, wherein when the frequency domain is to be searched, the control unit is configured to search the frequency domain by decreasing the oscillation frequency from a frequency higher than the frequency domain.

9. The fluid-property detection device according to Claim 1, further comprising a temperature sensor configured to measure a temperature of the inductor portion or a temperature of the fluid, wherein
   the control unit is configured to calculate a correction amount of a resonance frequency of the resonant circuit based on the temperature detected by the temperature sensor.

10. The fluid-property detection device according to Claim 1, further comprising an amplitude detector configured to detect an amplitude of the output signal, wherein
   when the amplitude becomes lower than a predetermined threshold value, the control unit is configured to search and detect a frequency domain in which the amplitude becomes equal to or higher than the threshold value by changing the oscillation frequency, and controls the oscillation frequency within the frequency domain.

FIG.1

FIG.2

FIG.3

FIG.4

EP 4 563 993 A1

I/O

R₁

3101

R₂

U₂

U₁

R₃

$Z = j\omega R_1 R_3 C_4 R_5 / R_2$

C₄

$L = R_1 R_3 C_4 R_5 / R_2$

R₅

FIG.5

FIG.6

```
                    ┌──────────────────────┐
                    │   Power Turned ON    │
                    └──────────────────────┘
                               │
                               ▼                      S701
┌─────────────────────────────────────────────────────────┐
│ Set oscillation frequency f of excitation signal to initial value (f_max) │
└─────────────────────────────────────────────────────────┘
                               │
                               ▼                      S702
┌─────────────────────────────────────────────────────────┐
│ Search frequency at which voltage V2 of resonant circuit exceeds │
│ Vr by lowering oscillation frequency f of excitation signal │
└─────────────────────────────────────────────────────────┘
                               │
                               ▼                      S703
┌─────────────────────────────────────────────────────────┐
│ Lower oscillation frequency until voltage V2 begins to decrease │
│ from its peak voltage │
└─────────────────────────────────────────────────────────┘
                               │
                               ▼                      S704
┌─────────────────────────────────────────────────────────┐
│ Calculate a plurality of index values θ1 of phase difference θ while │
│ scanning by gradually increasing and decreasing oscillation │
│ frequency f of excitation signal within frequency domain, in which │
│ phase difference becomes from θ0+θs to θ0-θs │
└─────────────────────────────────────────────────────────┘
                               │
                               ▼                      S705
┌─────────────────────────────────────────────────────────┐
│ Calculate regression equation linearly approximating relationship │
│ between oscillation frequency f and index value θ1 on coordinates │
└─────────────────────────────────────────────────────────┘
                               │
                               ▼                      S706
┌─────────────────────────────────────────────────────────┐
│ Calculate the monitor frequency fm at which the phase difference θ │
│ becomes θ0 from regression equation │
└─────────────────────────────────────────────────────────┘
                               │
                               ▼                      S707
YES ◄────────< Is the monitor frequency fm equal to or >──── NO
              < lower than upper limit frequency?        >
                               │
                               ▼                      S708
YES ◄────────< Is the monitor frequency fm equal to or >──── NO
              < higher than lower limit frequency?       >
                               │                            │
                               │                            ▼  S709
                               │                   ┌──────────────┐
                               │                   │ Warning signal │
                               │◄──────────────────│    output     │
                               ▼                   └──────────────┘
                                                      S710
YES ◄────────< Power supply ON? >──── NO ──► ┌──────┐
                               │              │ END  │
                               │              └──────┘
                               ▼                      S711
YES ◄────────< Is voltage V2 of resonant circuit equal to or >──── NO
              < higher than Vr?                              >
```

FIG.7

FIG.8

FIG.9

## INTERNATIONAL SEARCH REPORT

| International application No. |
| --- |
| **PCT/JP2023/027567** |

**A.    CLASSIFICATION OF SUBJECT MATTER**

**G01N 27/22**(2006.01)i; **G01N 27/00**(2006.01)i; **G01N 27/06**(2006.01)i
FI:    G01N27/22 B; G01N27/00 L; G01N27/06 B

According to International Patent Classification (IPC) or to both national classification and IPC

**B.    FIELDS SEARCHED**

Minimum documentation searched (classification system followed by classification symbols)

G01N27/00-G01N27/10; G01N27/14-G01N27/24; G01R27/00-G01R27/32

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 7-306172 A (MITSUBISHI ELECTRIC CORP) 21 November 1995 (1995-11-21) paragraphs [0034]-[0062], fig. 1-2 | 1, 6 |
| Y | | 3-4 |
| A | | 2, 5, 7-10 |
| Y | JP 10-163898 A (NIIGATA SEIMITSU KK) 19 June 1998 (1998-06-19) paragraphs [0034]-[0037], fig. 7 | 3-4 |
| A | WO 2009/050813 A1 (PIONEER CORPORATION) 23 April 2009 (2009-04-23) entire text | 1-10 |
| A | JP 6-066860 A (MURATA MANUFACTURING CO) 11 March 1994 (1994-03-11) entire text | 1-10 |
| A | US 2004/0036487 A1 (HEREMANS et al.) 26 February 2004 (2004-02-26) entire text | 1-10 |

☐ Further documents are listed in the continuation of Box C.    ☑ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **22 August 2023** | **05 September 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/027567**

| Patent document cited in search report | | | Publication date (day/month/year) | Patent family member(s) | | | Publication date (day/month/year) |
|---|---|---|---|---|---|---|---|
| JP | 7-306172 | A | 21 November 1995 | US | 5594163 | A | |
| | | | | column 9, line 35 to column 14, line 29, fig. 1-2 | | | |
| JP | 10-163898 | A | 19 June 1998 | (Family: none) | | | |
| WO | 2009/050813 | A1 | 23 April 2009 | US | 2010/0231239 | A1 | |
| JP | 6-066860 | A | 11 March 1994 | (Family: none) | | | |
| US | 2004/0036487 | A1 | 26 February 2004 | EP | 1391727 | A2 | |

Form PCT/ISA/210 (patent family annex) (January 2015)

**REFERENCES CITED IN THE DESCRIPTION**

**Patent documents cited in the description**

- JP 61170064 U

- JP 2022121000 A